# EUROPÄISCHE PATENTANMELDUNG

(11) **EP 1 323 469 A2**
(43) Veröffentlichungstag der Anmeldung: **02.07.2003**
(21) Anmeldenummer: 02022900.1
(22) Anmeldetag: 15.10.2002
(51) Int. Cl.: B01J 21/06, B01J 21/08, B01J 35/02, C07C 67/055

(54) **Trägerkatalysator**

(30) Priorität: 21.12.2001 DE 10163180
(71) Anmelder: Degussa AG, 40474 Düsseldorf (DE)
(72) Erfinder: Tacke, Thomas, Dr., 63755 Alzenau (DE); Krause, Helmfried, 63517 Rodenbach (DE); Lansink-Rotgerink, Hermanus Gerhardus Jozef, Dr., 63776 Mömbris-Mensengesäss (DE); Chen, Baoshu, Dr., Paducah, KY 42001 (US); Carson, Jason, Kirksey, KY 42054 (US)

(57) **Zusammenfassung**

Formkörper auf Basis von pyrogen hergestelltem Siliciumdioxid, gekennzeichnet durch eine ringförmige zylindrische Gestalt, dessen Querschnitt einen Hohlraum aufweist und wobei die Freifläche von 1,6 bis 77 % der Gesamtfläche des Querschnitts beträgt.

Trägerkatalysator, der auf einem Träger (Formkörper) als katalytisch aktive Komoponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, wobei daß es sich um einen Katalysator auf einem Träger mit einem Durchtrittskanal handelt, und die Aktivkomponenten eine Eindringtiefe von mehr als 0,5 mm bis 1,5 mm gerechnet von der Oberfläche des Trägermaterials, aufweisen.

Der Trägerkatalysator kann zur Herstellung von ungesättigten Estern aus Olefinen in der Gasphase eingesetzt werden.

## Beschreibung

Die Erfindung betrifft einen Trägerkatalysator, ein Verfahren zu seiner Herstellung sowie seine Verwendung, sowie Formkörper auf Basis von pyrogen hergestellten Oxiden. Insbesondere betrifft die Erfindung einen Katalysator auf Ringextrudaten zur Acetoxylierung von Olefinen, wie zum Beispiel die Herstellung von Vinylacetat Monomer nach dem Gasphasen-Verfahren.

Weiterhin betrifft die Erfindung Ringextrudate, ein Verfahren zu ihrer Herstellung sowie ihre Verwendung.

Verfahren zur Herstellung von Vinylacetatmonomer sind aus DE 16 68 088, EP-A 0 464 633, EP-A 0 519 435, EP-A 0 634 208, EP-A 0 723 810, EP-A 0 634 209, EP-A 0 632 214, EP-A 0 654 301, EP-A 0 723 810, US 4,048,096, US 5,185,308 und US 5,371,277 bekannt. Diese Dokumente beschreiben auch Verfahren zur Herstellung von Trägerkatalysatoren. Je nach Ausführungsform werden Trägerkatalysatoren mit homogener Edelmetallverteilung über den Trägerquerschnitt und mit mehr oder weniger ausgeprägtem Schalenprofil hergestellt.

Aus DE-B 21 00 778, US 4,902,823, US 5,250,487, US 5,292,931, US 5,808,136, EP 0 807 615, EP 0 916 402 und EP 0 987 058 ist es bekannt, Formkörper auf Basis pyrogen hergestellter Siliciumdioxide als Katalysatorträger bei der Herstellung von Vinylacetatmonomer einzusetzen.

EP-A 0 464 633 beschreibt einen Trägerkatalysator zur Herstellung von Vinylacetatemonomer auf Basis eines Katalysatorträgers mit mindestens einem Durchtrittskanal. Insbesondere wird auf einen Hohlzylinder verwiesen, bei dem sich mindestens 95% des Palladiums, Goldes und/oder deren Verbindungen in einem Bereich von der Oberfläche bis zu 0,5 mm unterhalb der Oberfläche des Trägers befinden. Derartige Trägerkatalysatoren weisen bei vermindertem Druckverlust über die Katalysatorschüttung eine ausreichende Aktivität und/oder Selektivität auf.

Eine Aufgabe der Erfindung ist es, einen Trägerkatalysator herzustellen, welcher bei gleicher beziehungsweise verbesserter Selektivität eine höhere Aktivität als bekannte Katalysatoren aufweist.

Gegenstand der Erfindung ist ein Trägerkatalysator, der auf einem Träger (Formkörper) als katalytisch aktive Komponenten Palladium und/oder dessen Verbindungen und Alkaliverbindungen sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, welcher dadurch gekennzeichnet ist, daß es sich um einen Katalysator auf einem Träger mit einem Durchtrittskanal handelt, und die Aktivkomponenten eine Eindringtiefe von mehr als 0,5 mm bis 1,5 mm, vorzugsweise mehr als 0,5 mm bis zu 1,0 mm, gerechnet von der Oberfläche des Trägermaterials, aufweisen.

Bevorzugt kann der Gegenstand der Erfindung ein Trägerkatalysator sein, der auf einem Träger (Formkörper) als katalytisch aktive Komoponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, welcher dadurch gekennzeichnet ist, daß es sich bei dem Träger um einen Hohlzylinder, eine Ringtablette, oder ein Ringextrudat mit einem Durchtrittskanal auf Basis von Siliziumdioxid, Alumosilikaten und anderen Rohstoffen handelt, die unter den Herstellbedingungen des Katalysatores und den Prozeßbedingungen zur Herstellung von Vinylactat Monomer mittels dem Gasphasen-Verfahren stabil sind.

Ein weiterer Gegenstand der Erfindung sind Formkörper, gekennzeichnet durch eine ringförmige zylindrische Gestalt, dessen Querschnitt einen Hohlraum aufweist und wobei die Freifläche von 1,6 bis 77 % der Gesamtfläche beträgt.

In einer Ausführungsform der Erfindung kann der Trägerkatalysator derart gestaltet sein, daß es sich bei dem Trägermaterial um einen Formkörper handelt, der aus einem oder mehreren pyrogenen Oxiden und/oder Mischoxiden aus der Reihe SiO₂, Al₂O₃, TiO₂ und ZrO₂ hergestellt wird.

Weiterhin kann der Trägerkatalysator derart gestaltet sein, daß es sich bei dem Trägermaterial um einen Formkörper handelt, der aus pyrogenen Mischoxiden hergestellt wird, wobei mindestens zwei Oxide aus der Gruppe SiO₂, Al₂O₃, TiO₂ und ZrO₂ verwendet werden.

In einer besonderen Ausführungsform der Erfindung kann es sich bei dem Trägermaterial um einen Formkörper handeln, der aus pyrogen hergestelltem Siliciumdioxid hergestellt wird, wobei der Formkörper die folgenden physikalisch-chemischen Kenndaten aufweist:

| | |
|---|---|
| Aussendurchmesser | 3 - 8 mm |
| Innendurchmesser | ≥ 1 mm |
| Anteil der Hohlraumfläche in % des Gesamtquerschnitts: | 1,6 - 77 % |
| BET-Oberfläche | 5 - 400 m²/g |

Die Formkörper auf Basis von pyrogenen Siliciumdioxid können gekennzeichnet sein durch eine ringförmige zylindrische Gestalt, dessen Querschnitt einen Hohlraum aufweist, und wobei die Freifläche von 1,6 bis 77 %, vorzugsweise 1,6 bis 56 %, der Gesamtfläche des Querschnitts beträgt.

Der Formkörper kann ein Hohlzylinder, eine Ringtablette und/oder ein Ringextrudat sein.

Als Alkaliverbindung können Kaliumverbindungen, wie zum Beispiel Kaliumacetat, bevorzugt eingesetzt werden.

Die katalytisch aktiven Komponenten können in folgenden Systemen vorhanden sein:
Pd/Au/Alkali-Verbindungen
Pd/Cd/Alkali-Verbindungen
Pd/Ba/Alkali-Verbindungen

In einer bevorzugten Ausführungsform der Erfindung kann der Trägerkatalysator als aktive Komponenten das System Pd/K/Au aufweisen.

Weitere Komponenten und/oder Promotoren zur Erhöhung der Katalysatoraktivität und/oder Katalysatorselektivität sind denkbar.

Die erfindungsgemäßen Trägerkatalysatoren können zur Herstellung ungesättigter Ester aus Olefinen, organischen Säuren und Sauerstoff in der Gasphase verwendet werden. Insbesondere können die erfindungsgemäßen Trägerkatalysatoren für die Produktion von Vinylacetatmonomer verwendet werden. Dazu werden Ethen, Essigsäure und molekularer Sauerstoff beziehungsweise Luft in der Gasphase, gegebenenfalls unter Zusatz von Inertgasen, bei Temperaturen zwischen 100 und 250 °C und bei normalem oder erhöhtem Druck, zum Beispiel 1 bis 25 bar, in Gegenwart des erfindungsgemäßen Trägerkatalysators zur Reaktion gebracht. Typischerweise werden Raumgeschwindigkeiten in Bezug auf die Gasphase von 1000 bis 5000 Liter Gasgemisch pro Liter Katalysator und pro Stunde realisiert.

Die erfindungsgemäßen Katalysatoren können auch zur Acetoxylierung von Olefinen, wie beispielsweise Propen, eingesetzt werden.

Ein weiterer Gegenstand der Erfindung ist ein Verfahren zur Herstellung des erfindungsgemäßen Trägerkatalysators durch Tränken, Aufsprühen, Aufdampfen, Tauchen oder Ausfällen der Pd-, Au, Cd-, Ba-Metallverbindungen, gegebenenfalls Reduzieren der auf dem Träger aufgebrachten reduzierbaren Metallverbindungen, Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, wobei der Träger ein Träger mit einem Durchtrittskanal ist. Der Träger kann ein Hohlzylinder, eine Ringtablette und/oder ein Ringextrudat sein.

Ein weiterer Gegenstand der Erfindung ist ein Verfahren zur Herstellung des erfindungsgemäßen Trägerkatalysators durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgen kann, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen insbesondere Kaliumacetat, die sich unter den Reaktionsbedingungen bei der Herstellung von Vinylacetatmonomer ganz oder teilweise in Alkaliacetate umwandeln, wobei der Träger einen Durchtrittskanal aufweist. Der Träger kann ein Hohlzylinder, eine Ringtablette und/oder ein Ringextrudat sein. Insbesondere kann der Träger ein Formkörper auf Basis von pyrogen hergestellten Ringextrudaten sein.

Bei Pd/Alkali/Ba-Katalysatoren können die Metallsalze durch bekannte Verfahren, wie Tränken, Aufsprühen, Aufdampfen, Tauchen oder Ausfällen aufgebracht werden (EP 0 519 436). Dieselben Methoden sind bei Pd/Alkali/Cd-Katalysatoren bekannt (US-PS 4,902,823, US-PS 3,393,199, US-PS 4,668,819).

Je nach Katalysatorsystem kann eine Reduktion des Trägerkatalysators vorgenommen werden.

Die Reduktion des Katalysators kann in der wäßrigen Phase oder in der Gasphase vorgenommen werden.

Zur Reduktion in der wäßrigen Phase eignen sich zum Beispiel Formaldehyd oder Hydrazin.

Die Reduktion in der Gasphase kann mit Wasserstoff, beziehungsweise Formiergas (95 Vol.-% N₂ + 5 Vol.-% H₂), Ethen oder stickstoffverdünntem Ethen vorgenommen werden.

Gemäß der EP 0 634 209 kann die Reduktion mit Wasserstoff bei Temperaturen zwischen 40 und 260 °C, bevorzugt zwischen 70 und 200 °C erfolgen.

Gemäß der EP-A 0 723 810 erfolgt die Reduktion mit Formiergas (95 Vol.-% N₂ und 5 Vol.-% H₂) bei Temperaturen zwischen 300 und 550 °C, bevorzugt zwischen 350 und 500 °C.

Bei dem Verfahren zur Herstellung von Vinylacetatmonomer wird der Katalysator erst langsam mit den Reaktanden belastet. Während dieser Anfahrphase erhöht sich die Aktivität des Katalysators und erreicht gewöhnlich erst nach Tagen oder Wochen ihr endgültiges Niveau.

Wichtig ist, daß die Katalysatorträger unter den Reaktionsbedingungen des katalytischen Prozesses, insbesondere unter dem Einfluß von Essigsäure, ihre mechanische Festigkeit behalten.

Der erfindungsgemäße Trägerkatalysator erreicht auf Grund einer gesteigerten Aktivität und/oder einer verbesserten Selektivität eine deutlich verbesserte Produktausbeute.

Im folgenden wird die Herstellung der erfindungsgemäßen Trägerkatalysatoren am Beispiel des Systems Pd/Alkali/Au näher beschrieben.

Formkörper, insbesondere in der Form von Hohlzylindern (Ringextrudate) werden gemäß einer Ausführungsform der Erfindung mit einer Palladium und Gold enthaltenden Lösung imprägniert. Gleichzeitig mit der edelmetallhaltigen Lösung oder in beliebiger Reihenfolge nacheinander können die eingesetzten Trägermaterialien mit einer basischen Lösung, welche ein oder mehrere basische Verbindungen enthalten kann, imprägniert werden. Die basische Verbindung oder Verbindungen dienen zur Überführung des Palladiums und Golds in ihre Hydroxide.

Die Verbindungen in der basischen Lösung können aus Alkalihydroxiden, Alkalibicarbonaten, Alkalicarbonaten, Alkalisilikaten oder Mischungen dieser Stoffe bestehen. Bevorzugt können Kaliumhydroxid, Natriumhydroxid und/oder Natriummetasilikat verwendet werden.

Zur Herstellung der edelmetallhaltigen Lösung können als Palladiumsalze, beispielsweise Palladiumchlorid, Natriumbeziehungsweise Kaliumpalladiumchlorid oder Palladiumnitrat, verwendet werden.

Als Goldsalze eignen sich Gold(III)-chlorid und Tetrachlorogold(III)-säure.

Vorzugsweise können Kaliumpalladiumchlorid, Natriumpalladiumchlorid und/oder Tetrachlorogoldsäure eingesetzt werden.

Das Imprägnieren des Katalysatorträgers mit der basischen Lösung beeinflußt die Abscheidung der Edelmetalle auf dem Katalysatorträger. Die basische Lösung kann entweder gleichzeitig mit der Edelmetallösung oder in beliebiger Reihenfolge mit dieser Lösung mit dem Katalysatorträger in Kontakt gebracht werden. Bei aufeinander-folgender Imprägnierung des Katalysatorträgers mit den beiden Lösungen kann nach dem ersten Imprägnierschritt eine Zwischentrocknung vorgenommen werden.

Bevorzugt kann der Katalysatorträger zuerst mit der basischen Verbindung imprägniert werden. Die anschließende Imprägnierung mit der Palladium und Gold enthaltenden Lösung kann zur Ausfällung von Palladium und Gold in einer oberflächlichen Schale auf dem Katalysatorträger führen. Die umgekehrte Reihenfolge der Imprägnierungen kann im allgemeinen zu einer mehr oder weniger homogenen Verteilung der Edelmetalle über den Querschnitt des Katalysatorträger führen. Bei geeigneter Verfahrensführung können jedoch auch bei umgekehrter Imprägnier-Reihenfolge Katalysatoren mit definierter Schale erhalten werden (US 4,048,096). Katalysatoren mit homogener oder nahezu homogener Edelmetall-Verteilung können im allgemeinen eine geringere Aktivität und Selektivität aufweisen.

Die Schalendicke kann durch die Menge der auf das Trägermaterial aufgebrachten basischen Verbindung relativ zur gewünschten Menge der Edelmetalle beeinflußt werden. Je höher dieses Verhältnis ist, desto geringer wird die Dicke der sich ausbildenden Schale. Das für eine gewünschte Schalendicke erforderliche Mengenverhältnis von basischer Verbindung zu den Edelmetallverbindungen kann von der Beschaffenheit des Trägermaterials sowie von der gewählten basischen Verbindung und den Edelmetallverbindungen abhängen. Das erforderliche Mengenverhältnis wird zweckmäßigerweise durch wenige Vorversuche ermittelt. Die sich ergebende Schalendicke kann dabei in einfacher Weise durch Aufschneiden der Katalysatorteilchen ermittelt werden.

Die minimal notwendige Menge der basischen Verbindung ergibt sich aus der stöchiometrisch berechneten Menge an Hydroxidionen, die zur Überführung des Palladiums und des Golds in die Hydroxide benötigt werden. Als Richtwert gilt, daß die basische Verbindung für eine Schalendicke von bis zu 1,0 mm in einem 1 bis 10-fachen stöchiometrischen Überschuß angewandt werden sollte.

Katalysatorträger können nach dem Verfahren der Porenvolumenimprägnierung mit den basischen Verbindungen und den Edelmetallsalzen belegt werden. Wird mit Zwischentrocknung gearbeitet, wählt man die Volumina der beiden Lösungen so, daß sie jeweils etwa 90 bis 100 % der Aufnahmekapazität der Katalysatorträger entsprechen. Wird auf die Zwischentrocknung verzichtet, so muß die Summe der Einzelvolumina der beiden Imprägnierlösungen der obigen Bedingung entsprechen, wobei die Einzelvolumina im Verhältnis von 1 : 9 bis 9 : 1 zueinander stehen können. Bevorzugt wird ein Volumenverhältnis von 3 : 7 bis 7 : 3, insbesondere von 1 : 1, angewendet. Als Lösungsmittel kann in beiden Fällen bevorzugt Wasser verwendet werden. Es können aber auch geeignete organische oder wäßrigorganische Lösungsmittel eingesetzt werden.

Die Umsetzung der Edelmetallsalzlösung mit der basischen Lösung zu unlöslichen Edelmetallverbindungen kann langsam erfolgen und ist je nach Präparationsmethode im allgemeinen erst nach 1 bis 24 Stunden abgeschlossen. Danach werden die wasserunlöslichen Edelmetallverbindungen mit Reduktionsmitteln behandelt. Es kann eine Naßreduktion, zum Beispiel mit wäßrigem Hydrazinhydrat, oder eine Gasphasenreduktion mit Wasserstoff, Ethen, oder Formiergas vorgenommen werden. Die Reduktion kann bei Normaltemperatur oder erhöhter Temperatur und bei Normaldruck oder erhöhtem Druck, gegebenenfalls auch unter Zugabe von Inertgasen, wie zum Beispiel Stickstoff, erfolgen.

Vor und/oder nach der Reduktion der Edelmetallverbindungen kann das auf dem Träger gegebenenfalls vorhandene Chlorid durch eine gründliche Waschung entfernt werden. Nach der Waschung kann der Katalysator weniger als 500, vorzugsweise weniger als 200 ppm, Chlorid enthalten.

Die nach der Reduktion erhaltene Katalysatorvorstufe kann getrocknet und abschließend mit Alkaliacetaten oder Alkaliverbindungen, die sich unter den Reaktionsbedingungen bei der Produktion von Vinylacetatmonomer ganz oder teilweise in Alkaliacetate umwandeln, imprägniert werden. Vorzugsweise kann mit Kaliumacetat imprägniert werden. Hierbei kann wieder bevorzugt die Porenvolumenimprägnierung angewandt werden. Das heißt: Die benötigte Menge an Kaliumacetat wird in einem Lösungsmittel, vorzugsweise Wasser, dessen Volumen etwa der Aufnahmekapazität des vorgelegten Trägermaterials für das gewählte Lösungsmittel entspricht, gelöst. Dieses Volumen ist etwa gleich dem Gesamtporenvolumen des Trägermaterials.

Der fertige Katalysator kann anschießend bis auf eine Restfeuchte von weniger als 5 % getrocknet werden. Die Trocknung kann an der Luft, gegebenenfalls auch unter Stickstoff als Inertgas, erfolgen.

Die Herstellung von Trägerkatalysatoren der Systeme Pd/Alkali/Cd beziehungsweise Pd/Alkali/Ba auf geeigneten Trägermaterialien kann nach den oben zitierten Patentschriften erfolgen.

Für die Synthese von Vinylacetatmonomer ist es zweckmäßig, den Katalysator mit 0,2 bis 4, vorzugsweise 0,3 bis 3 Gew.-% Palladium, 0,1 bis 2, vorzugsweise 0,15 bis 1,5 Gew.-% Gold und 1 bis 10, vorzugsweise 1,5 bis 9 Gew.-% Kaliumacetat, jeweils bezogen auf das Gewicht des eingesetzten Trägers, zu belegen. Diese Angaben gelten für das System Pd/Alkali/Au.

Im Fall von Katalysatorträgern mit einer Schüttdichte von 500 g/l entsprechen diese Konzentrationsangaben volumenbezogenen Konzentrationen von 1,0 bis 20 g/l Palladium, 0,5 bis 10 g/l Gold und 5 bis 50 g/l Kaliumacetat.

Zur Anfertigung der Imprägnierlösungen können die entsprechenden Mengen der Palladium- und Goldverbindungen in einem Volumen Wasser, welches etwa 10 bis 100 % der Wasseraufnahmekapazität des vorgelegten Trägermaterials entspricht, gelöst werden. Ebenso kann bei der Anfertigung der basischen Lösung verfahren werden.

Der Cadmium-Gehalt der Pd/Alkali/Cd-Katalysatoren kann 0,1 bis 2,5 Gew.-%, vorzugsweise 0,4 bis 2,0 Gew.-% betragen.

Der Barium-Gehalt der Pd/Alkali/Ba-Katalysatoren kann 0,1 bis 2,0 Gew.-%, vorzugsweise 0,2 bis 1,8 Gew.-% betragen.

Der Palladium-Gehalt der Pd/Alkali/Cd beziehungsweise Pd/Alkali/Ba-Katalysatoren kann 0,2 bis 4 Gew.-%, bevorzugt 0,3 bis 3 Gew.-% Palladium, betragen.

Der Kalium-Gehalt der Pd/Alkali/Cd- bzw. Pd/Alkali/Ba-Katalysatoren kann 1 bis 10 Gew.-%, vorzugsweise 1,5 bis 9 Gew.-% betragen.

In einer besonderen Ausführungsform dieser Erfindung kann ein Trägermaterial auf Basis von aus pyrogen hergestelltem Siliciumdioxid hergestellten Ringextrudaten mit den folgenden physikalisch-chemischen Kenndaten eingesetzt werden:

| | |
|---|---|
| Aussendurchmesser | 3 - 8 mm |
| Innendurchmesser | ≥ 1 mm |
| Anteil der Hohlraumfläche in % des Gesamtquerschnitts: | 1,6 - 77 % |
| BET-Oberfläche | 5 - 400 m²/g |

Das Verfahren zur Herstellung dieser bevorzugten Katalysatorträger ist dadurch gekennzeichnet , daß man pyrogen hergestelltes Siliciumdioxid mit Methylhydroxypropylcellulose und/oder, Wachsemulsion und/oder Polysaccharid unter Zusatz von schwach ammoniakalischem Wasser kompaktiert, gegebenenfalls zur Verbesserung der Gleitfähigkeit der Masse bei der Extrusion Polyethylenoxid zusetzt, die Masse extrudiert, bei einer Temperatur von 30 - 150 °C trocknet und die erhaltenen Ringextrudate während eines Zeitraumes von 0,5 - 8 Stunden bei einer Temperatur von 400 - 1200 °C tempert. Zur Durchführung sind alle Mischer und Kneter, die ein gute Homogenisierung ermöglichen, wie zum Beispiel Pflugscharmischer, Intensivmischer, Sigma-Kneter, Z-Kneter, geeignet.

Die Herstellung der Formkörper kann auf Stempelpressen, Ein- oder Zweischnecken-Extrudern erfolgen.

Vor der Formgebung kann in einer besonderen Ausführungsform Masse mit Wasser hergestellt werden, die folgende Zusammensetzung aufweisen:

| | |
|---|---|
| 70 - 98 Gew.-% | Siliciumdioxid, vorzugsweise 85 - 95 Gew.-% |
| 0,1 - 20 Gew.-% | Methylhydroxypropylcellulose, vorzugsweise 1 - 10 Gew.-% |
| 0,1 - 20 Gew.-% | Wachsemulsion, vorzugsweise 2 - 10 Gew.-% |
| 0,1 - 5 Gew.-% | Polysaccharid, vorzugsweise 0,2 - 1,5 Gew.-% |
| 0,1 - 5 Gew.-% | Polyethylenoxid, vorzugsweise 0,2 - 2 Gew.-% |

Die Ringextrudate können Formen mit folgenden Abmessungen aufweisen:

| | |
|---|---|
| Aussendurchmesser | 3 - 8 mm |
| Innendurchmesser | ≥1 mm |
| Anteil der Hohlraumfläche in % des Gesamtquerschnitts: | 1,6 - 77 % |

Die Ringextrudate können bei 400 - 1200 °C 30 Min. bis 8 Stunden getempert werden. Nach der Temperung können sie danach als Katalysatorträger eingesetzt werden.

Durch Variation der Einsatzstoffe und der Temperatur der Temperung können Bruchfestigkeit, Porenvolumen und spezifische Oberfläche eingestellt werden.

Als pyrogen hergestelltes Siliciumdioxid können Siliciumdioxide mit den physikalisch-chemischen Kenndaten gemäß Tabelle 1 eingesetzt werden:

**Tabelle 1:**

| Prüfmethode | | Aerosil 90 | Aerosil 130 | Aerosil 150 | Aerosil 200 | Aerosil 300 | Aerosil 380 |
|---|---|---|---|---|---|---|---|
| Verhalten gegenüber Wasser | hydrophil | | | | | | |
| Aussehen | lockeres weißes Pulver | | | | | | |
| Oberfläche nach BET¹ | m²/g | 90 ± 15 | 130 ± 25 | 150 ± 15 | 200 ± 25 | 300 ± 30 | 380 ± 30 |
| Mittlere Größe der Primärteilchen | nm | 20 | 16 | 14 | 12 | 7 | 7 |
| Stampfdichte (ca.-Wert)² | g/l | 80 | 50 | 50 | 50 | 50 | 50 |
| Trocknungsverlust³ (2 Std. bei 105 °C) bei Verlassen des Lieferwerkes | % | < 1,0 | < 1,5 | < 0,5⁹⁾ | < 1,5 | < 1,5 | < 2,0 |
| Gluhverlust^{4 7} 2 Stunden bei 1000 °C) | % | < 1 | < 1 | < 1 | < 1 | < 2 | < 2,5 |
| pH-Wert⁵ | 3,7-4,7 | 3,7-4,7 | 3,7-4,7 | 3,7-4,7 | 3,7-4,7 | 3,7-4,7 | 3,7-4,7 |
| SiO₂⁸ | % | > 99,8 | > 99,8 | > 99,8 | > 99,8 | > 99,8 | > 99,8 |
| Al₂O₃⁸ | % | < 0,05 | < 0,05 | < 0,05 | < 0,05 | < 0,05 | < 0,05 |
| Fe₂O₃⁸ | % | < 0,003 | < 0,003 | < 0,003 | < 0,003 | < 0,003 | < 0,003 |
| TiO₂⁸ | % | < 0,03 | < 0,03 | < 0,03 | < 0,03 | < 0,03 | < 0,03 |
| HCI ⁸ ¹⁰ | % | < 0,025 | < 0,025 | < 0,025 | < 0,025 | < 0,025 | < 0,025 |
| Siebrückstand⁶ (nach Mocker,45 mm) | % | < 0,05 | < 0,05 | < 0,05 | < 0,05 | < 0,05 | < 0,05 |

| | | | | | | | |
|---|---|---|---|---|---|---|---|
| ¹ in Anlehnung and DIN 66131 | | | | | | | |
| ² in Anlehnung an DIN ISO 787/XI, JIS K 5101/18 (nicht gesiebt) | | | | | | | |
| ³ in Anlehnung an DIN ISO 787/II, ASTM D 280, JIS K 5101/21 | | | | | | | |
| ⁴ in Anlehnung an DIN 55921, ASTM D 1208, JIS K 5101/23 | | | | | | | |
| ⁵ in Anlehnung an DIN ISO 787/IX, ASTM D 1208, JIS K 5101/24 | | | | | | | |
| ⁷ bezogen auf die 2 Stunden bei 105 °C getrocknete Substanz | | | | | | | |
| ⁸ bezogen auf die 2 Stunden bei 1000 °C geglühte Substanz ⁹ spezielle vor Feuchtigkeit schützende Verpackung | | | | | | | |
| ¹⁰ HCI-Gehalt ist Bestandteil des Glühverlustes | | | | | | | |

Zur Herstellung des pyrogen hergestellten Siliciumdioxides (z. B. AEROSIL® der Firma Degussa) wird eine Knallgasflamme aus Wasserstoff und Luft eine flüchtige Siliciumverbindung eingedüst. In den meisten Fällen verwendet man Siliciumtetrachlorid. Diese Substanz hydrolysiert unter dem Einfluß des bei der Knallgasreaktion entstehenden Wassers zu Siliciumdioxid und Salzsäure. Das Siliciumdioxid tritt nach dem Verlassen der Flamme in eine sogenannte Koagulationszone ein, in der die AEROSIL®-Primärteilchen und-Primäraggregate agglomerieren. Das in diesem Stadium als eine Art Aerosol vorliegende Produkt wird in Zyklonen von den gasförmigen Begleitsubstanzen und anschliessend mit feuchter Heissluft nachbehandelt. Durch dieses Verfahren lässt sich der Rest-Salzsäuregehalt unter 0,025 % senken. Da das AEROSIL® am Ende dieses Prozesses mit einer Schüttdichte von nur ca. 15 g/l anfällt, wird eine Vakuumverdichtung, mit der sich Stampfdichten von ca. 50 g/l und mehr einstellen lassen, angeschlossen.

Die Teilchengrössen der auf diese Weise gewonnenen Produkte können mit Hilfe der Reaktionsbedingungen, wie zum Beispiel die Flammentemperatur, der Wasserstoff- oder Sauerstoffanteil, die Siliciumtetrachloridmenge, die Verweilzeit in der Flamme oder die Länge der Koagulationsstrecke, variiert werden.

Die BET-Oberfläche wird gemäß DIN 66 131 mit Stickstoff bestimmt.

Das Porenvolumen wird rechnerich aus der Summe von Mikro-, Meso- und Makroporenvolumen bestimmt.

Die Bruchfestigkeit wird mittels des Bruchfestigkeitstesters der Firma Erweka, Typ TBH 28, bestimmt.

Die Bestimmung der Mikro- und Mesoporen erfolgt durch Aufnahme einer N₂-Isotherme und deren Auswertung nach BET, de Boer und Barret, Joyner, Halenda.

Die Bestimmung der Makroporen erfolgt durch das Hg-Einpressverfahren.

Der Abrieb wird mittels des Abrieb- und Friabilitätstesters der Firma Erweka, Typ TAR, bestimmt.

### Beispiel 1

| | |
|---|---|
| 94,3 Gew.-% | Aerosil® 380 |
| 1,9 Gew.-% | Methylhydroxypropylcellulose |
| 3,3 Gew.-% | Wachsemulsion |
| 0,5 Gew.-% | Polysaccharid |

werden unter Zusatz von Wasser, welches mit einer wässrigen ammoniakalischen Lösung (12 ml einer 25 %igen Lösung für einen 1 Kg Ansatz) schwach alkalisch gestellt wird, in einem Mischer kompaktiert. Diese Masse wird in einem Einschneckenextruder zu Ringextrudaten geformt und mit einer Schneidvorrichtung auf die gewünschte Länge geschnitten. Die Formkörper werden in einem Bandtrockner bei 120 °C getrocknet und anschließend 3 Stunden bei 900 °C calciniert.

### Beispiel 2

| | |
|---|---|
| 93,0 Gew.-% | Aerosil® 200 |
| 3,25 Gew.-% | Methylhydroxypropylcellulose |
| 9,25 Gew.-% | Wachsemulsion |
| 0,5 Gew.-% | Polysaccharid |

werden unter Zusatz von Wasser, welches mit einer wässrigen ammoniakalischen Lösung (12 ml einer 25 %igen Lösung für einen 1 Kg Ansatz) schwach alkalisch gestellt wird, in einem Mischer kompaktiert. Die Masse wird in einem Einschneckenextruder zu Ringextrudaten geformt und mit einer Schneidvorrichtung auf die gewünschte Länge geschnitten. Die Formkörper werden in einem Bandtrockner bei 120 °C getrocknet und anschliessend 3 Stunden bei 900 °C calciniert.

### Beispiel 3

Es werden Ringextrudate, wie in Beispiel 2 beschrieben, hergestellt. Jedoch erfolgt die Calzinierung 3 Stunden bei 850 °C.

### Beispiel 4

| | |
|---|---|
| 93,0 Gew.-% | Aerosil® 380 |
| 3,25 Gew.-% | Methylhydroxypropylcellulose |
| 3,25 Gew.-% | Wachsemulsion |
| 0,5 Gew.-% | Polysaccharid |

werden unter Zusatz von Wasser, welches mit einer wässrigen ammoniakalischen Lösung (12 ml einer 25 %igen Lösung für einen 1 Kg Ansatz) schwach alkalisch gestellt wird, in einem Mischer kompaktiert. Die Masse wird in einem Einschneckenextruder zu Ringextrudaten geformt und mit einer Schneidvorrichtung auf die gewünschte Länge geschnitten. Die Formkörper werden in einem Bandtrockner bei 120 °C getrocknet und anschließend 3 Stunden bei 900 °C calciniert.

### Beispiel 5

Es werden Ringextrudate, wie Beispiel 4 beschrieben, hergestellt. Jedoch erfolgt die Calcinierung bei 850 °C.

### Beispiel 6

| | |
|---|---|
| 93,0 Gew.-% | Aerosil® 380 |
| 2,8 Gew.-% | Methylhydroxypropylcellulose |
| 2,3 Gew.-% | Wachsemulsion |
| 1,4 Gew.-% | Polyethylenoxid |
| 0,5 Gew.-% | Polysaccharid |

werden unter Zusatz von Wasser, welches mit einer wässrigen ammoniakalischen Lösung (12 ml einer 25 %igen Lösung für einen 1 Kg Ansatz) schwach alkalisch gestellt wird, in einem Mischer kompaktiert. Die Masse wird in einem Einschneckenextruder zu Ringextrudaten geformt und mit einer Schneidvorrichtung auf die gewünschte Länge geschnitten. Die Formkörper werden in einem Bandtrockner bei 120 °C getrocknet und anschließend 3 Stunden bei 850 °C calciniert.

**Tabelle 2**

| Beispiel | 1 | 2 | 3 | 4 | 5 | 6 |
|---|---|---|---|---|---|---|
| Bruchfestigkeit (N) | 24 | 27 | 24 | 44 | 34 | 24 |
| | | | | | | |
| Abrieb (wt%) | 2,25 | 3,75 | 5,2 | 1,5 | 2,5 | 5,3 |
| | | | | | | |
| Wasserporenvolumen (ml/g) | 0,62 | 0,90 | 1,02 | 0,65 | 0,81 | 0,94 |
| | | | | | | |
| Stampfdichte g/l | n.b. | 375 | 340 | 465 | 400 | 315 |
| | | | | | | |
| Durchmesser (mm) | | | | | | |
| Aussen | 5,02 | 5,3 | 5,4 | 4,6 | 4,8 | 5,7 |
| Innen | 2,8 | 2,2 | 2,3 | 1,9 | 2,0 | 3,0 |
| Anteil Hohlraum-fläche am Gesamtquer-schnitt (%) | 31 | 17 | 18 | 17 | 17 | 28 |
| | | | | | | |
| Spez. Oberfläche m²/g | n.b. | 140 | 163 | 157 | 192 | n.b. |

Die erfindungsgemäßen Formkörper zeigen beziehungsweise ermöglichen:
◆ niedrige Druckverluste
◆ niedrige Schüttdichten
◆ relativ große äußere Oberflächen pro Einheitsvolumen eines Reaktionsgefäßes
◆ einen guten Stoff- und Wärmetransport
◆ und insbesondere im Vergleich zu bekannten Hohlzylindern und anderen Trägerformen, wie z. B. auch wabenförmigen Trägermaterialien, eine deutlich erhöhte Bruch- und Abriebsfestigkeit.

In den folgenden Beispielen wird die Leistungsfähigkeit des erfindungsgemäßen Trägerkatalysators auf Basis von Hohlzylindern (Ringextrudaten) erläutert. Insbesondere wird hierbei auf Ringextrudate auf Basis von pyrogen hergestellten Oxiden eingegangen.

Die Katalysatoren werden unter anderem durch Puls-Chemisorption mit Kohlenmonoxid hinsichtlich ihrer Edelmetalldispersion charakterisiert. Hierbei kommt das Gerät der Firma Micromeritics (Modell Nr. 2910) zum Einsatz. Da es sich bei den Katalysatoren um bimetallische Systeme handelt, wird auf die Angabe der Dispersion in Prozent verzichtet. Anstelle dessen wird die reine Aufnahme von Kohlenmonoxid durch Puls-Chemisorption ermittelt und als Vergleich für die Dispersion der Katalysatoren herangezogen.

### Beispiel 7 (Vergleichsbeispiel)

Es wird ein Palladium-Gold-Kaliumacetat-Katalysator auf Basis von Beispiel 1 gemäß EP-A 0 464 633 hergestellt. Als Katalysatorträger wird ein Träger gemäß Beispiel 5 eingesetzt. Die Konzentration der Imprägnierlösungen wird so gewählt, daß der fertige Katalysator eine Konzentration von 0,55 Gew.-% Palladium, 0,25 Gew.-% Gold und 5,0 Gew.-% Kaliumacetat enthält. Der Katalysator weist eine CO-Aufnahme, gemessen durch Puls Chemisorption, von 0,108 ml/g und eine Schalendicke in Bezug auf Palladium und Gold von 0,1 mm auf.

Dies ist der nicht-erfindungsgemäße Katalysator A.

### Beispiel 8 (Vergleichsbeispiel)

Es wird ein Palladium-Gold-Kaliumacetat-Katalysator auf Basis von Beispiel 1 gemäß EP-A 0 464 633 hergestellt. Als Katalysatorträger wird ein Träger gemäß Beispiel 5 eingesetzt. Die Konzentration der Imprägnierlösungen und der Herstellprozeß werden so angepasst, daß der fertige Katalysator eine Konzentration von 0,55 Gew.-% Palladium, 0,25 Gew.-% Gold und 5,0 Gew.-% Kaliumacetat enthält und eine vollständige Eindringtiefe in Bezug auf Palladium und Gold aufweist. Der Katalysator weist eine CO-Aufnahme, gemessen durch Puls Chemisorption, von 0,212 ml/g.

Dies ist der nicht-erfindungsgemäße Katalysator B.

### Beispiel 9

Es wird ein Palladium-Gold-Kaliumacetat-Katalysator auf Basis des Katalysatorträgers gemäß Beispiel 5 hergestellt. Die Konzentration der Imprägnierlösungen wird so gewählt, daß der fertige Katalysator eine Konzentration von 0,55 Gew.-% Palladium, 0,25 Gew.-% Gold und 5,0 Gew.-% Kaliumacetat enthält.

In einem ersten Schritt wird der Träger zunächst mit einer basischen Lösung aus Natriumhydroxid in Wasser imprägniert. Das Volumen der wäßrigen NaOH-Lösung entspricht 50 Prozent der Wasseraufnahme des trockenen Trägers. Nach der Imprägnierung mit Natriumhydroxid wird der Träger unmittelbar ohne Zwischentrocknung mit einer wäßrigen Edelmetalllösung aus Natriumpalladiumchlorid und Tetrachlorogoldsäure imprägniert, deren Volumen ebenfalls 50 Prozent der Wasseraufnahmekapazität des trockenen Trägermaterials entspricht.

Nach einer Wartezeit von 1,5 Stunden zwecks Hydrolyse der Edelmetallverbindungen werden die Trägerteilchen chloridfrei gewaschen. Der Katalysator wird getrocknet und bei 450 °C in der Gasphase mit Formiergas reduziert. Danach wird der Katalysator mit einer wäßrigen Kaliumacetat-Lösung imprägniert und erneut getrocknet. Die Trocknung wird in der Gasphase mit Luft durchgeführt.

Die Konzentration der basischen Lösung an Natriumhydroxid ist so bemessen, daß sich auf den Trägerteilchen eine edelmetallhaltige Schale von < 1.0 mm ausbildet. Der Katalysator weist eine CO-Aufnahme, gemessen durch Puls Chemisorption, von 0,178 ml/g und eine Schalendicke in Bezug auf Palladium und Gold von mehr als 0,6 mm auf, wobei 0,8 mm jedoch nicht überschritten werden. Dieser Katalysator enthält ebenfalls 0,55 Gew.-% Palladium, 0,25 Gew.-% Gold und 5,0 Gew.-% Kaliumacetat.

Dies ist der erfindungsgemäße Katalysator C.

### Beispiel 10

Die nicht-erfindungsgemäßen Katalysatoren A und B (Beispiele 7 und 8) sowie der erfindungsgemäße Katalysator C (Beispiel 9) werden in einem Leistungstest bei der Herstellung von Vinylacetatmonomer (VAM) untersucht.

Die Aktivität und Selektivität der Katalysatoren sowie die Ausbeute an Vinylacetatmonomer werden während einer Prüfung von bis zu 80 Stunden in einem Screeningtest gemessen.

Dazu werden jeweils 20,0 ml der einzelnen Katalysatoren nachdem sie mit Inertmaterial verdünnt werden in einem mit Öl auf 150 Grad Celsius beheizten Strömungsrohrreaktor (Reaktorlänge 710 mm, Innendurchmesser 23,7 mm) bei 4 bar Überdruck und einer Raumgeschwindigkeit (GHSV) von 5000 h⁻¹ mit der folgenden Gaszusammensetzung geprüft: 66,0 Vol.-% Ethen, 18.0 Vol.-% Essigsäure, 6.0 Vol.-% Sauerstoff und 10.0 Vol.-% Stickstoff.

Die Reaktionsprodukte werden im Ausgang des Reaktors mittels Gaschromatographie on-line analysiert. Zur Beurteilung der einzelnen Katalysatoren wird die Ausbeute an Vinylacetatmonomer in Prozent herangezogen, wobei die Werte normiert werden und der Katalysator A nach Beispiel 7 auf 100 Prozent gesetzt wird. Katalysator A entspricht dem Stand der Technik gemäß EP 0 464 633 A.

Die Testergebnisse sind in der Tabelle 3 zusammengefaßt.

**Tabelle 3**

| Katalysator | Relative VAM Ausbeute in [%] | Katalysatortemperatur [°C] |
|---|---|---|
| A (nicht erfindungsgemäß) | 100 | 154,8 |
| B (nicht erfindungsgemäß) | 75.1 | 155,3 |
| C (erfindungsgemäß) | 123,0 | 155,8 |

Der Katalysator A (Ringextrudate mit einer Schalendicke in Bezug auf Palladium und Gold von weniger als 0,5 mm) weist eine deutlich höhere Ausbeute an Vinylacetatmonomer als der Katalysator B nach Beispiel 8 auf.

Der Katalysator B weist eine homogene Verteilung von Palladium und Gold über den Trägerquerschnitt auf. Damit sind die Ergebnisse aus EP 0 464 633 bestätigt.

Überraschenderweise zeigt sich jedoch, daß der erfindungsgemäße Katalysator C nach Beispiel 9 mit einer Schalendicke von 0,5 bis 1,0 mm in Bezug auf Palladium und Gold die höchste Ausbeute an Vinylacetatmonomer aufweist.

## Patentansprüche

1. Trägerkatalysator, der auf einem Träger (Formkörper) als katalytisch aktive Komoponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, **dadurch gekennzeichnet, daß** es sich um einen Katalysator auf einem Träger mit einem Durchtrittskanal handelt, und die Aktivkomponenten eine Eindringtiefe von mehr als 0,5 mm bis 1,5 mm, gerechnet von der Oberfläche des Trägermaterials, aufweisen.

2. Trägerkatalysator nach Anspruch 1, **dadurch gekennzeichnet, daß** es sich um einen Katalysator auf einem Träger mit einem Durchtrittskanal handelt, und die Aktivkomponenten eine Eindringtiefe von mehr als 0,5 bis zu 1,0 mm, gerechnet von der Oberfläche des Trägermaterials, aufweisen.

3. Trägerkatalysator gemäß einem der Ansprüche 1 bis 2, **dadurch gekennzeichnet, daß** es sich bei dem Trägermaterial um einen Formkörper handelt, der aus pyrogen hergestelltem Siliciumdioxid hergestellt wird, wobei der Formkörper die folgenden physikalisch-chemischen Kenngrößen aufweist:
| | |
|---|---|
| Aussendurchmesser | 3 - 8 mm |
| Innendurchmesser | ≥ 1 mm |
| Anteil Hohlraumfläce in % | |
| des Gesamtquerschnitts: | 1,6 - 77 % |
| BET-Oberfläche | 5 - 400 m²/g |

4. Trägerkatalysator gemäß einem der Ansprüche 1 bis 2, **dadurch gekennzeichnet, daß** es sich bei dem Trägermaterial um einen Formkörper handelt, der aus einem oder mehreren pyrogenen Oxiden aus der Reihe SiO₂, Al₂O₃, TiO₂ und ZrO₂ hergestellt wird.

5. Trägerkatalysator gemäß einem der Ansprüche 1 bis 2, **dadurch gekennzeichnet, daß** es sich bei dem Trägermaterial um einen Formkörper handelt, der aus pyrogenen Mischoxiden hergestellt wird, wobei mindestens zwei Oxide aus der Gruppe SiO₂, Al₂O₃, TiO₂ und ZrO₂ verwendet werden.

6. Trägerkatalysator gemäß einem der Ansprüche 1 bis 5, **dadurch gekennzeichnet, daß** als aktive Komponenten das System Pd/K/Au verwendet wird.

7. Trägerkatalysator gemäß einem der Ansprüche 1 bis 6, **dadurch gekennzeichnet, daß** es sich bei der Alkaliverbindung um Kaliumacetat handelt.

8. Verfahren zur Herstellung des Trägerkatalysators gemäß den Ansprüchen 1 bis 7 durch Tränken, Aufsprühen, Aufdampfen, Tauchen oder Ausfällen der Pd-, Au, Cd-, Ba-Metallverbindungen, gegebenenfalls Reduzieren der auf dem Träger aufgebrachten reduzierbaren Metallverbindungen, Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, **dadurch gekennzeichnet, daß** der Katalysator ein Trägerkatalysator nach einem der Ansprüche 1 bis 7 ist.

9. Verfahren zur Herstellung des Trägerkatalysators gemäß den Ansprüchen 1 bis 8 durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Herstellung von Vinylacetatmonomer ganz oder teilweise in Alkaliacetate umwandeln, **dadurch gekennzeichnet, daß** der Katalysator ein Trägerkatalysator nach einem der Ansprüche 1 bis 8 ist.

10. Verfahren nach Anspruch 8 oder 9, **dadurch gekennzeichnet, daß** es sich bei dem Alkaliacetat beziehungsweise der Alkaliverbindung um Kaliumacetat handelt.

11. Verwendung eines Trägerkatalysators gemäß den Ansprüchen 1 bis 7 zur Herstellung ungesättigter Ester aus Olefinen, organischen Säuren und Sauerstoff in der Gasphase.

12. Verwendung eines Trägerkatalysators gemäß einem oder mehreren der Ansprüche 1 bis 11 zur Herstellung von Vinylacetatmonomer.

13. Formkörper, **gekennzeichnet durch** eine ringförmige zylindrische Gestalt, dessen Querschnitt einen Hohlraum aufweist und wobei die Freifläche von 1,6 bis 77 % der Gesamtfläche beträgt.

14. Formkörper, der aus einem oder mehreren pyrogenen Oxiden aus der Reihe SiO₂, Al₂O₃ und ZrO₂ hergestellt wird, **gekennzeichnet durch** eine ringförmige zylindrische Gestalt, dessen Querschnitt einen Hohlraum aufweist und wobei die Freifläche von 1,6 bis 77 % der Gesamtfläche beträgt.

15. Formkörper gemäß Anspruch 13, **dadurch gekennzeichnet, daß** der Formkörper aus pyrogenen Mischoxiden hergestellt wird, wobei mindestens zwei Oxide aus der Gruppe SiO₂, Al₂O₃, TiO₂ und ZrO₂ verwendet werden.

16. Formkörper, gemäß Anspruch 13, **dadurch gekennzeichnet, daß** der Formkörper die folgenden physikalisch-chemischen Kenndaten aufweist:
| | |
|---|---|
| Aussendurchmesser | 3 - 8 mm |
| Innendurchmesser | ≥ 1 mm |
| Anteil Hohlraumfläche in % des Gesamtquerschnitts: | 1,6 - 77 % |
| BET-Oberfläche | 5 - 400 m²/g |

17. Formkörper, gemäß Anspruch 13, **dadurch gekennzeichnet, daß** er auf Basis von pyrogen hergestelltem Siliciumdioxid hergestellt und eine ringförmige zylindrische Gestalt aufweist, dessen Querschnitt einen Hohlraum aufweist und wobei die Freifläche von 1,6 bis 77 % der Gesamtfläche beträgt.

18. Formkörper, gemäß Anspruch 13 der aus pyrogen hergestelltem Siliciumdioxid hergestellt wird, wobei der Formkörper die folgenden physikalisch-chemischen Kenndaten aufweist:
| | |
|---|---|
| Aussendurchmesser | 3 - 8 mm |
| Innendurchmesser | ≥ 1 mm |
| Anteil Hohlraumfläche in % des Gesamtquerschnitts: | 1,6 - 77 % |
| BET-Oberfläche | 5 - 400 m²/g |
